Europäisches Patentamt

European Patent Office

Office européen des brevets

(19)

(11) Publication number: **0 174 585**
A2

(12) # EUROPEAN PATENT APPLICATION

(21) Application number: **85111081.7**

(22) Date of filing: **03.09.85**

(51) Int. Cl.⁴: **C 12 N 15/00**
**C 12 P 21/02, C 12 N 1/18**

(30) Priority: **14.09.84 US 650936**

(43) Date of publication of application:
**19.03.86 Bulletin 86/12**

(84) Designated Contracting States:
**AT CH DE FR GB IT LI NL SE**

(71) Applicant: **MILES LABORATORIES, INC.**
**1127 Myrtle Street**
**Elkhart Indiana 46514(US)**

(72) Inventor: **Yarger, James Gregory**
**1539 Evergreen Place**
**Elkhart Indiana 46515(US)**

(74) Representative: **Dänner, Klaus, Dr. et al,**
**c/o Bayer AG Konzernverwaltung RP Patentabteilung**
**D-5090 Leverkusen 1 Bayerwerk(DE)**

(54) **Promoters and the use thereof in the expression of unfused procaryotic or eucaryotic proteins in yeast.**

(57) A modified *GAL7* promoter having a unique restriction site inserted in the transcriptional leader sequence prior to an ATG translation initiation codon is disclosed. Also disclosed is a hybrid gene and a method of use thereof for the expression of unfused procaryotic or eucaryotic proteins in yeast.

EP 0 174 585 A2

Croydon Printing Company Ltd.

## PROMOTERS AND THE USE THEREOF IN THE EXPRESSION OF UNFUSED PROCARYOTIC OR EUCARYOTIC PROTEINS IN YEAST

### BACKGROUND OF THE INVENTION

The present invention is directed to a new form of yeast GAL7 promoter. Current methodologies provide for the expression of cloned genes by use of fusion protein technology in which the cloned gene is inserted after the ATG translation initiation sequence. This is undesirable in that the correct reading frame can be difficult to obtain for every cloned gene, and often expression results in a fusion protein which is generally undesirable for commercial applications. The modified GAL7 promoter, when used to prepare a hybrid gene as described herein avoids these disadvantages and allows the expression of a cloned gene without concern for proper reading frame of the cloned gene and without the production of fusion proteins.

### DESCRIPTION OF PERTINENT ART

Gene, 24, 289-297 (1983) describes the fusion of the proinsulin gene with the promoter and

MS-1355

protein leader sequence of the GAL1 gene (coding for yeast galactokinase) in the yeast-*E. coli* plasmid vector pYT 7810. The proinsulin gene was fused in correct reading frame at two different sites within the GAL1 coding sequence and fused hybrid polypeptides containing proinsulin were expressed in *Saccharomyces cerevisiae*. The method described by the authors suffers from the disadvantage of expressing a fusion protein which requires the proinsulin to be cleaved from the galactokinase leader polypeptide by treatment with cyanogen bromide.

St. John and Davis, <u>J. Mol. Biol.</u>, 152, 285-315 (1981) describe the detailed structural and transcriptional features of the <u>GAL7-GAL10-GAL1</u> gene cluster in *Saccharomyces*. St. John et al, <u>J. Mol. Biol.</u>, 152, 317-334 (1981) further describe the deletion analysis of the <u>GAL</u> gene cluster and the cloning of various deletions into the shuttle vector YRp15.

U.S. Patent 4,332,892 teaches the use of a "portable promoter" in the expression of an unfused polypeptide in bacteria. The portable promoter consists of a DNA fragment containing a transcription initiation site recognized by RNA polymerase but containing no protein translational start site. The promoter is inserted ahead of the protein translational start site of the desired gene thereby producing a fused gene having a hybrid ribosomal binding site.

MS-1355

None of the references cited above describe or suggest the modified eucaryotic GAL7 promoter of the present invention nor the use thereof in the expression of unfused procaryotic or eucaryotic protein in yeast.

## SUMMARY OF THE INVENTION

The present invention is directed to a modified GAL7 promoter having inserted within the transcriptional leader sequence a unique XhoI restriction site, said restriction site inserted prior to an ATG translation initiation codon for the GAL7 coding region. Also disclosed is a hybrid gene capable of expressing unfused procaryotic or eucaryotic protein. Said hybrid gene contains (in sequence): (1) a GAL7 promoter having an upstream activation site and a XhoI restriction site within the transcriptional leader sequence inserted prior to an ATG translation initiation codon; (2) fused thereto at said XhoI restriction site a gene coding for said procaryotic or eucaryotic protein; and (3) a transcription terminator composed of 450 base pairs of the GAL10 carboxyl terminus. Also disclosed is a method of producing unfused procaryotic or eucaryotic protein in yeast. The method includes the steps of (1) inserting into a yeast shuttle plasmid the hybrid gene described above; (2) inserting said plasmid into said yeast to transform the yeast with said plasmid; (3) culturing said transformed yeast to produce said

MS-1355

procaryotic or eucaryotic protein; and (4) recovering from said yeast said procaryotic or eucaryotic protein.

While the restriction site described herein in the transcriptional leader sequence is identified as a XhoI restriction site, it is to be understood that any restriction site may be inserted within said leader sequence using the techniques disclosed herein. Reference herein to such a restriction site as a XhoI restriction site is merely exemplary and is done solely for the sake of clarity and readability.

## DETAILED DESCRIPTION OF THE INVENTION

The present invention concerns the construction of a new form of the *Saccharomyces* GAL7 promoter. The modified GAL7 promoter has inserted within the transcriptional leader sequence a unique XhoI restriction site. Said GAL7 promoter may have ligated thereto (at said XhoI restriction site) the gene coding for the desired procaryotic or eucaryotic protein to be expressed or alternatively may be used to prepare a hybrid gene as described hereinafter. Said hybrid gene allows for the expression of a cloned gene without the concomitant reading frame problems and without the production of a fusion protein often encountered by known methodologies. Details of the construction of the unique XhoI restriction site in the GAL7 promoter as well as the preparation and use of the hybrid gene are provided hereinafter.

The GAL7 promoter which is modified as described hereinafter was subcloned from isolates provided by St. John and Davis on a 1.7 kb SalI to HindIII fragment (the HindIII site being subsequently changed to a BamHI site using BamHI linkers according to standard procedures as described hereafter). Said promoter includes an upstream activation site contained within an approximately 125 base pair region beginning 180 base pairs upstream of the GAL7 ATG translational start site. Because of this upstream activation site, transcription is induced only by galactose. It is believed that the upstream activation site may serve as an entry site for RNA polymerase which then initiates transcription downstream of the TATA box (a sequence required for transcription initiation from the GAL7 promoter).

Present within the modified GAL7 promoter described herein is a unique XhoI restriction site inserted within the transcriptional leader sequence (approximately 22 base pairs in length) and just prior to the translation initiation codon of the coding region of the GAL7 gene. The XhoI restriction site is produced in the transcriptional leader sequence by modifications of standard M13 mutagenesis techniques such as described in Nucleic Acids Research, 11: 5103-5112 (1983) or Nucleic Acids Research, 12: 4011-4017 (1984) which are incorporated herein by reference. Preferably the restriction site is a XhoI restriction site, however, using commercially available linkers any unique restriction site can be produced in the modified GAL7 promoter of the invention in place of the XhoI site.

The hybrid gene of the present invention includes a gene coding for the desired procaryotic or eucaryotic protein cloned into the XhoI restriction site of the modified GAL7 promoter (without the GAL7 coding region) described above utilizing restriction endonuclease digestions followed by ligation of the desired gene at the XhoI site with DNA ligase. These manipulations are carried out using techniques which are well known in the art and well within the capabilities of the skilled artisan. The genes of interest may be any which code for a procaryotic or eucaryotic protein such as human serum albumin, human insulin, human blood coagulation factors, aprotinin, calf chymosin, human gonadotropin, xylose isomerase, and the like.

The hybrid gene also includes a transcription terminator from the carboxyl terminus of the adjacent GAL10 gene inserted at the end of the coding region of the gene of interest. The terminator contains about 450 base pairs of DNA located on a BamHI fragment derived from the carboxyl terminus of the adjacent GAL10 gene.

The hybrid gene of this invention is used for the production of protein in the following manner. The modified GAL7 promoter having fused thereto (at the XhoI site) the gene of interest coding for a procaryotic or eucaryotic protein followed by the transcription terminator described above is inserted into a yeast shuttle plasmid using conventional techniques. Suitable yeast shuttle plasmids include the following: pJY6, YEp13, YEp24, YCp19 (available from the American Type Culture Collection) and the like. The resulting recombinant expression vector is then used to transform the desired yeast host (typically a strain of *Saccharomyces cerevisiae*) which transformants may then be cultured to produce and express the unfused procaryotic or eucaryotic protein followed by recovery of said protein.

The following example is provided as a means of illustrating the present invention and is not to be construed as a limitation thereon.

The modified GAL7 promoter was prepared as follows. The 1.7 kilobase SalI to BamHI fragment from plasmid pJY6 containing the GAL7 promoter and 575 base pairs of the GAL7 coding region was ligated into the SalI and BamHI sites of phage vector M13mP9 according to standard M13 cloning procedures to yield the single stranded M13mP9-GAL7 vector. Plasmid pJY6 (a yeast shuttle vector containing the yeast URA3 gene and the yeast 2µ replicon, with the 1.7 kilobase SalI to BamHI GAL7 promoter ligated into the BamHI and SalI sites of the pBR322 region of the vector) has been deposited with the American Type Culture Collection and has been assigned ATCC accession number 39851. Vector

MS-1355

M13mP9 is commercially available to the public from sources such as New England Biolabs.

The transcriptional leader sequence of the GAL7 gene (cloned as above into M13mP9) has the following partial sequence (sequence A):

A)    5'-AAACAGTTGAATATTCCCTCAAAA ATG ACT-3'

B)                  3'-AAGGGAGCTCT TAC TGA-5'

The 17 base, double mismatch primer shown above (i.e., 3'-AAGGGAGCTCTTACTGA-5', sequence B) was used to produce the unique XhoI site (CTCGAG) in the GAL7 transcriptional leader sequence of the M13mP9-GAL7 single stranded, closed, circular DNA vector by the following procedure.

1 microliter (μl) of 10X kinase labeling buffer (0.5M tris pH8.0; 0.1M MgCl$_2$; 0.05M dithiothreitol; 4% glycerol; 1mM spermidine), 1μl of the double mismatch primer (1μg or 160 pmoles), 6μl of γ$^{32}$ P-adenosine triphosphate (12 μCi), and 2μl kinase (New England Biolabs, 2 units/μl) were incubated together at 37°C for 20 minutes. Then 1μl of 10mM adenosine triphosphate was added and the entire mixture was incubated for an additional 10 minutes at 37°C to yield the kinased double mismatch primer.

1μl (16 pmoles) of the kinased, double mismatch primer described in the above paragraph was mixed with 1μl of universal primer (20 pmoles), 3μl of single stranded M13mP9-GAL7 DNA vector (0.6 pmoles) and 25μl of TMND buffer (40mM tris, pH 7.5;

MS-1355

100 m$\underline{M}$ Nacl; 20m$\underline{M}$ MgCl$_2$; and 1m$\underline{M}$ dithiothreitol). This mixture was incubated for 30 seconds at 85°C, then for 5 minutes at 55°C and finally for 20 minutes at 23°C. To this was added 1µl of Klenow enzyme (5 units), 0.5 µl T4DNA ligase, and 8µl of the following: 3m$\underline{M}$ dithiothreitol; 188µ$\underline{M}$ each of dATP, dGTP, dCTP and dTTP; and 188µ$\underline{M}$ of adenosine triphosphate. The entire mixture was incubated for 1 hour at 20°C after which 1.0µl of T4DNA ligase and 3µl of 10m$\underline{M}$ adenosine triphosphate were added and the mixture was incubated for an additional 1 hour at 20°C.

The incubation mixture was extracted with phenol and the aqueous DNA-containing fraction after ethanol precipitation was resuspended in 50µl of 1x $\underline{BamHI}$ buffer (150m$\underline{M}$ NaCl; 6m$\underline{M}$ tris-HCL, pH7.9; 6m$\underline{M}$ MgCl$_2$; and 100µg/ml bovine serum albumin). The DNA was digested with 16 units of $\underline{BamHI}$ and 15 units of $\underline{SalI}$ restriction endonucleases for 2 hours at 37°C. The 1.7 kilobase $\underline{SalI}$ to $\underline{BamHI}$ fragment containing the $\underline{GAL7}$ fragment was gel-isolated from 1 percent agarose and ligated into the $\underline{SalI}$ and $\underline{BamHI}$ sites of plasmid pBR322 according to standard procedures and transformed into *E. coli* strain RR1 which was subsequently grown and cultured. Plasmid pBR322 and *E. coli* strain RR1 are commercially available from the American Type Culture Collection.

In order to locate clones containing the $\underline{XhoI}$ site, the 1·g plasmid DNA from the above colonies was digested with 10 units and 16 units,

MS-1355

respectively, of XhoI and BamHI restriction enzymes in 1x BamHI buffer (described above). Clones were identified which contained a 575 base pair XhoI to BamHI fragment from the GAL7 coding region. Approximately 1 out of 25 clones contained the desired XhoI site by this procedure (these clones are now referred to as pJY6-X). Plasmid pJY6-X has been deposited with the American Type Culture Collection and has been assigned ATCC accession number 39852. Those clones (i.e., pJY6-X) can now be treated with SalI and BamHI restriction endonucleases as previously described to obtain the modified GAL7 DNA fragment which now contains the desired XhoI restriction site in the transcriptional leader sequence (i.e., a modified GAL7 promoter). This SalI to BamHI fragment containing the XhoI site can be ligated back into plasmid pJY6 (which is then referred to as pJY6-YX) or any other desired yeast shuttle vector such as YEp24 as previously described.

The hybrid gene utilizing the modified GAL7 promoter is constructed as follows. The calf chymosin gene located on a 1.01 kilobase PstI to BclI DNA fragment and containing its own ATG translation initiation codon is modified by adding XhoI linkers to the PstI site by standard procedures. See, for example, Maniatis et al, Molecular Cloning: A Laboratory Manual, Cold Spring Harbor Press, Cold Spring Harbor, NY (1982), which is incorporated herein by reference. The XhoI to BclI fragment carrying the chymosin gene is now cloned into the XhoI and BamHI sites of the modified GAL7 promoter present on plasmid pJY6-YX. The gene thus formed is referred to as pJY6-YX-chymosin. A transcription terminator containing 450 base pairs of DNA and located on a BamHI fragment derived from the carboxyl terminus of the adjacent GAL10 gene is then ligated into the unique BamHI site of plasmid pJY6-YX-chymosin.

The GAL10 terminator was derived from plasmid pJY6 in the following manner. The NcoI restriction site located approximately 350 base pairs downstream from the carboxyl terminus of the GAL10 gene was changed to a BamHI site by adding a BamHI linker by standard procedures such as described in Molecular Cloning: A Laboratory Manual, cited supra. The TaqI restriction site located approximately 100 base pairs within the terminus of the GAL10 coding region was changed to a BamHI site by again adding a BamHI linker as described above. This construction resulted in a transcription terminator 450 base pairs in length which is bounded by BamHI restriction sites and which functions efficiently in either orientation. The plasmid containing this transcription terminator is referred to as plasmid pPT4.3 which has been deposited with the American Type Culture Collection and has been assigned accession number 39850. This transcription terminator, which may be permanently fixed by blunt-end ligations at the BamHI site or left portable by sticky-end ligations at the BamHI site, serves to help eliminate run-on transcription beyond the chymosin gene (or any other gene of interest).

The hybrid gene is used to produce unfused proteins as follows. The plasmid containing the hybrid gene (in this case pJY6-YX-chymosin) is used to transform *Saccharomyces cerevisiae* (strain DBy745). Transformants are selected utilizing conventional techniques (i.e,. RIA-screening techniques to identify those transformants producing chymosin), and are then cultured by conventional methodologies under conditions suitable for production of the chymosin. Similarly, recovery of the chymosin is by standard methods said chymosin being unfused (i.e., free from any extraneous peptides).

In the case of certain cloned genes, for example rat growth hormone (<u>Nature</u> 293: 717-722, 1981), the first 10 base pairs preceding the ATG translational start codon are not efficiently recognized in yeast to promote translation initiation. In other instances a cloned gene may lack all or a part of the ATG translation start codon. Plasmid pJY6-X allows for the simple modification and efficient expression of such genes by the following technique.

MS-1355

A piece of synthetic DNA of variable length is synthesized by standard procedures and contains, in part, the sequence:

```
5'-TCGAGAAAAACTATAATG.....
        CTTTTTGATATTAC.......
   |____|____|_____|_____|
     A      B       C
```

This synthetic DNA contains, in order, a 5' overhang with homology to a XhoI restriction site (A), a GAL1-like transcriptional leader sequence ending in an ATG codon (B), and a DNA sequence corresponding to the coding region for the gene of interest and ending in any desired restriction site within the gene to be expressed (C). The synthetic DNA is then ligated between the XhoI site of pJY6-YX and the selected restriction site within the gene to be expressed thereby producing substantially increased gene expression. This procedure also provides one with a simple method to choose between expressing genes containing the pro- or pre-pro- regions of said proteins or limiting expression to the mature form of the protein.

While the disclosure herein describes the construction and use of a modified GAL7 promoter for preparing hybrid genes and the use of such hybrid genes in the production of unfused, procaryotic or eucaryotic proteins the skilled artisan can readily appreciate that the same techniques can be applied to the GAL1 and GAL10 promoters of

*Saccharomyces*. Accordingly, GAL1 and GAL10 promoters modified and used as described herein are deemed to be equivalents of the modified GAL7 promoter and uses thereof as taught in this specification. Also, GAL7 promoters (as well as GAL1 and GAL10 promoters) modified to contain a restriction site other than the XhoI restriction site in the approximately 22 base pair transcriptional leader sequence are deemed to be equivalent to the modified GAL7 promoter taught herein containing said XhoI site in said transcriptional leader sequence.

WHAT IS CLAIMED IS:

1.  A modified GAL7 promoter comprising a GAL7 promoter having inserted within the transcriptional leader sequence a unique restriction site, said restriction site inserted prior to an ATG translation initiation codon of the coding region of the GAL7 gene.

2.  The promoter of Claim 1 wherein said restriction site is a XhoI restriction site.

3.  A hybrid gene capable of expressing unfused procaryotic or eucaryotic proteins comprising in the following sequence (1) a GAL7 promoter having an upstream activation site, and a restriction site within the transcriptional leader sequence said restriction site inserted prior to an ATG translation initiation codon; (2) fused thereto a gene coding for said procaryotic or eucaryotic protein; and (3) a transcription terminator composed of 450 base pairs of the GAL10 carboxyl terminus.

4.  The hybrid gene of Claim 3 wherein said restriction site is a XhoI restriction site.

5.  A yeast cell containing the hybrid gene of Claim 3 said cell being capable of producing an unfused procaryotic or eucaryotic protein.

MS-1355

6. A method of producing unfused procaryotic or eucaryotic proteins in yeast which comprises the steps of (1) inserting into a yeast shuttle plasmid the hybrid gene of claim 3; (2) inserting said plasmid into said yeast to transform the yeast with said plasmid; (3) culturing said transformed yeast to produce said procaryotic or eucaryotic protein; and (4) recovering from said yeast said procaryotic or eucaryotic protein.

MS-1355

7.   A method of efficiently expressing in yeast cloned genes which are otherwise either not expressed or only poorly expressed which comprises the steps of:

(1)   inserting into the hybrid gene of claim 3 a length of synthetic DNA containing in sequence:

   (a)   a <u>XhoI</u> homologous 5' overhang,

   (b)   about a 10 base pair region of <u>GAL1</u> leader sequence DNA, and

   (c)   a length of DNA homologous to said cloned gene;

(2)   inserting into said yeast the gene construct of (1) above thereby transforming said yeast with said gene construct;

(3)   culturing said transformed yeast to produce an unfused procaryotic or eucaryotic protein encoded by said cloned gene; and

(4)   recovering from said yeast said unfused procaryotic or eucaryotic protein.

MS-1355